# EUROPEAN PATENT SPECIFICATION

(11) **EP 3 541 774 B1**
(45) Date of publication and mention of the grant of the patent: **30.11.2022**
(21) Application number: 17798238.6
(22) Date of filing: 16.11.2017
(51) Int. Cl.: C07C 45/45, C07C 49/203, C07C 49/21

(54) **NOVEL USE OF PHENYL PHOSPHINIC ACID**
NEUARTIGE VERWENDUNG VON PHENYLPHOSPHINSÄURE
NOUVELLE UTILISATION D'ACIDE PHÉNYLPHOSPHINIQUE

(30) Priority: 18.11.2016 EP 16199444
(43) Date of publication of application: 25.09.2019
(73) Proprietor: DSM IP Assets B.V., 6411 TE Heerlen (NL)
(72) Inventor: AQUINO, Fabrice, 4303 Kaiseraugst (CH); BONRATH, Werner, 4303 Kaiseraugst (CH); RIEBEL, Peter Hans, 4303 Kaiseraugst (CH)
(74) Representative: Dux, Roland
(86) International application number: PCT/EP2017/079526
(87) International publication number: WO 2018/091624

(56) References cited:
- EP-A2- 2 346 805
- US-A- 6 034 279

## Description

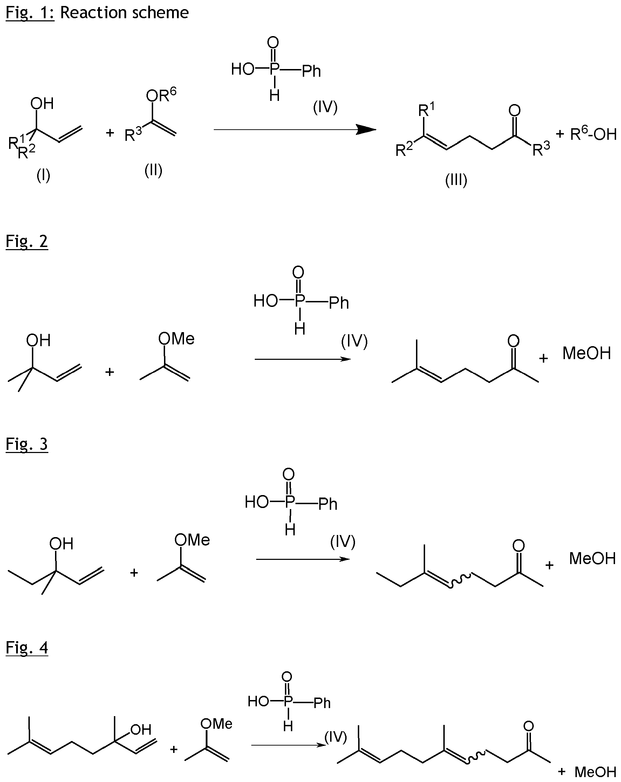

The present invention is directed towards an industrial sustainable process for the manufacture of gamma,delta-unsaturated ketones of formula (III) in the presence of phenylphosphinic acid (see formula (IV)) as catalyst, 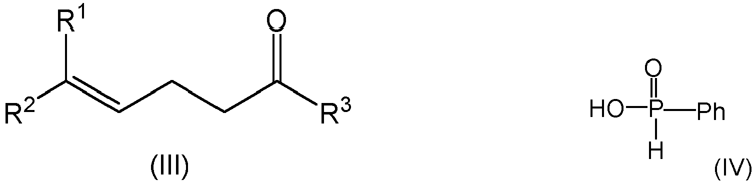 wherein R¹ is methyl or ethyl, R³ is methyl,
R² is a saturated or unsaturated linear, branched or cyclic hydrocarbyl group with 1 to 46 C atoms (see also **Fig. 1****).**

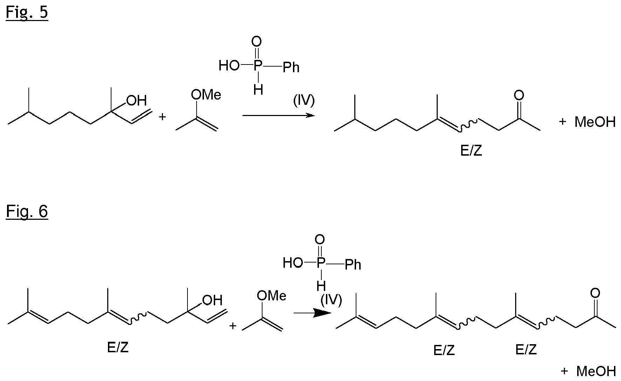

The present invention is especially directed towards an industrial sustainable process for the manufacture of 6-methyl-5-hepten-2-one ("MH"; see **Fig. 2**), 6-methyl-5-octen-2-one ("EH"; see **Fig. 3**), 6,10-dimethyl-5,9-undecadien-2-one (= geranylacetone; "GA"; see **Fig. 4**), 6,10-dimethyl-5-undecen-2-one (= dihydrogeranylacetone; "DHGA"; see **Fig. 5**) and 6,10,14-trimethyl-5,9,13-pentadeca-trien-2-one (= farnesylacetone; "FA"; see **Fig. 6**).

US6034279 describes the preparation of gamma, delta-unsaturated ketones from a tertiary allyl alcohol and alkenyl alkyl ether in the presence of diphenylphosphinic acid as catalyst.

### Detailed description

The present invention is directed towards a process for the manufacture of gamma,delta-unsaturated ketones of the general formula (III) 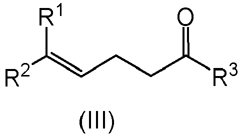 by reacting a tertiary vinyl carbinol of the general formula (I) with an isopropenyl alkyl ether of the general formula (II) in the presence of a catalyst of the general formula (IV), 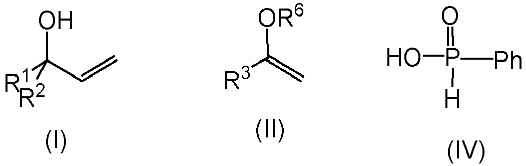 wherein R¹ and R⁶ are independently from each other methyl or ethyl, R³ is methyl, and R² is a saturated or unsaturated linear, branched or cyclic hydrocarbyl group with 1 to 46 C atoms.

### Starting materials

### Compound of the formula (I)

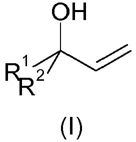

R¹ is either methyl or ethyl, and R² is a saturated or unsaturated linear, branched or cyclic hydrocarbyl group with 1 to 46 C atoms.

Preferred examples of R² are listed in **table 1.**

**Table 1:**

| **R²** | |
|---|---|
| methyl | CH₃ |
| ethyl | CH₂CH₃ |
| 4-methyl-pentyl | 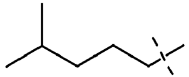 |
| CH₂-prenyl = "homoprenyl" | 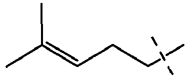 |
| CH₂-geranyl/CH₂-neryl = "homogeranyl"/" homoneryl" | 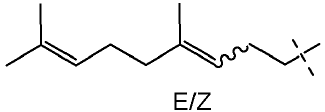 |
| CH₂-farnesyl = "homofarnesyl" | 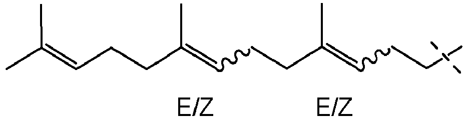 |
| CH₂-hexahydrofarnesyl | 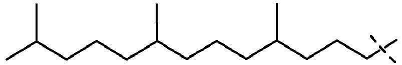 |
| CH₂-solanesyl = "homosolanesyl" | 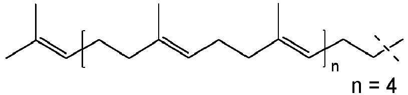 |
| CH=CH-(2,6,6-trimethylcyclohex-1 | 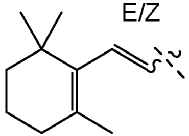 |

The compound of the general formula (I) is preferably selected from the group consisting of 2-methyl-3-buten-2-ol ("MBE"), 3-methyl-4-penten-3-ol ("EBE"), 3,7-dimethyl-1,6-octadien-3-ol (= linalool, "LL"), 3,7-dimethyl-1-octen-3-ol ("DMOE"), and (6E)-3,7,11-trimethyl-1,6-dodecadien-3-ol (= E-nerolidol, "E-NL"). The compound of formula (I) is more preferred MBE or EBE.

### Compound of the formula (II)

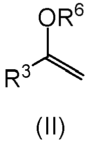

The compound of formula (II) is preferably either isopropenyl methyl ether ("IPM"; R³ = methyl) or isopropenyl ethyl ether ("IPE"; R³ = ethyl), whereby isopropenyl methyl ether is preferred.

### Amounts of compounds of the formula (I) and (II)

The molar ratio of the tertiary vinyl carbinol of the general formula (I) to the isopropenyl alkyl ether of the general formula (II) is preferably ranging from 1:7 to 1:2, more preferably ranging from 1:5 to 1:1.5, most preferably ranging from 1:3.5 to 1:2.

### Catalyst of the formula (IV)

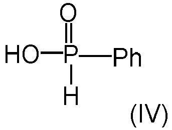

The amount of the catalyst of formula (IV), "PIAc" (phenyl phosphinic acid), is preferably ranging from 0.01-0.3 mol-%, more preferably ranging from 0.02-0.25 mol-%, most preferably ranging from 0.1-0.25 mol-%, based on the amount of the tertiary vinyl carbinol of the general formula (I).

### Reaction conditions

### Temperature

The reaction is preferably carried out at a temperature ranging from 100 to 170°C, more preferably at a temperature ranging from 110 to 160°C, most preferably at a temperature ranging from 120 to 150° C.

### Pressure

The reaction is preferably carried out at a pressure ranging from 5 to 15 bar, more preferably at a pressure ranging from 8 to 12 bar.

### Solvent

The reaction can be carried out without solvent or in the presence of an organic solvent. Preferably the reaction is carried out without solvent.

Even if the reaction is carried out in the absence of an organic solvent, the starting materials, the compounds of formula (I) and (II), as well as the catalyst of formula (IV) may still be provided in an organic solvent. Thus, there may be an amount of organic solvent up to 10 weight-%, preferably an amount of organic solvent up to 5 weight-%, more preferably an amount of organic solvent up to 3 weight-%, based on the total weight of the reaction mixture.

If the reaction is carried out in an organic solvent, polar aprotic organic solvents such as aliphatic ketones as e.g. acetone are preferred.

The reaction mixture itself, with or without an organic solvent, is also an object of the present invention. Thus, the present invention is directed towards a reaction mixture comprising a compound of formula (I), a compound of formula (II) and a catalyst of formula (IV), as well as to a reaction mixture consisting essentially of a compound of formula (I), a compound of formula (II) and a catalyst of formula (IV) 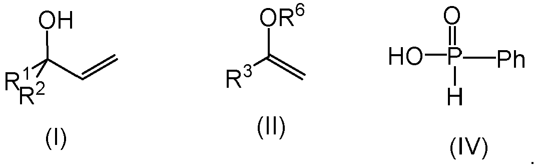 "consisting essentially of" in this context means that the total amount of the compound of formula (I), the compound of formula (II) and the catalyst of formula (IV) sum up to an amount of at least 90 weight-%, preferably of at least 95 weight-%, more preferably of at least 97 weight-%, based on the total amount of the reaction mixture.

The present invention is also directed towards the novel use of a catalyst of the formula (IV) 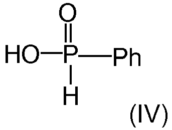 for the manufacture of gamma,delta-unsaturated ketones of the general formula (III) 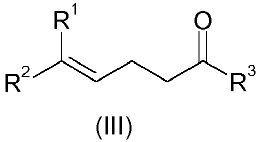 by reacting a tertiary vinyl carbinol of the general formula (I) with an isopropenyl alkyl ether of the general formula (II) 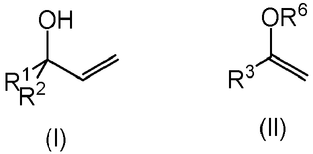 wherein R¹ and R⁶ are independently from each other methyl or ethyl, R³ is methyl, and
R² is a saturated or unsaturated linear, branched or cyclic hydrocarbyl group with 1 to 46 C atoms, whereby the preferences as given above also apply.

The present invention is now further illustrated by the following non-limiting examples.

### Examples:

### General procedure

The powderous catalyst PIAc (in the amounts as given in the tables below) is filled into the reactor as such. 144 g of the starting material MBE and the amount of IPM ("eq." refers to the molar amount of the other starting material, the compound of formula (I)) as given in the tables below are mixed and poured into the reactor. The reactor is closed and evacuated three times with nitrogen. The reaction mixture is stirred and heated up to the temperature as given in the tables below. The experiment is then carried out at isothermal conditions. After the reaction time given in the tables, the reaction mixture is cooled down to 20°C. A sample of the reaction mixture is collected and directly neutralized with sodium acetate. The sample is then analyzed by gas chromatography.

**Table 2: Conditions: 2.1 eq. IPM/150°C**

| Example | catalyst | Amount of catalyst [mol-%], based on MBE | Conversion MBE [%] | Yield MH [%] |
|---|---|---|---|---|
| 1 | PIAc | 0.24 | 97.6 | 91.1 |

**Table 3: Conditions: 3 eq. IPM/150°C**

| Example | catalyst | Amount of catalyst [mol-%], based on MBE | Conversion MBE [%] | Yield MH [%] | Selectivity [%] |
|---|---|---|---|---|---|
| 2 | PIAc | 0.25 | 99.6 | 93.2 | 94 |

## Claims

1. A process for the manufacture of gamma,delta-unsaturated ketones of the general formula (III) 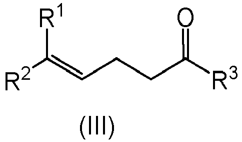 by reacting a tertiary vinyl carbinol of the general formula (I) with an isopropenyl alkyl ether of the general formula (II) in the presence of a catalyst of the general formula (IV), 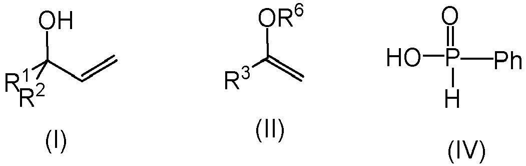 wherein R¹ and R⁶ are independently from each other methyl or ethyl, R³ is methyl, and R² is a saturated or unsaturated linear, branched or cyclic hydrocarbyl group with 1 to 46 C atoms.

2. The process according to claim 1, wherein the reaction is carried out at a temperature ranging from 100 to 170°C, preferably at a temperature ranging from 110 to 160°C, more preferably at a temperature ranging from 120 to 150°C.

3. The process according to any one or more of the preceding claims,
wherein the reaction is carried out at a pressure ranging from 5 to 15 bar, preferably at a pressure ranging from 8 to 12 bar.

4. The process according to any one or more of the preceding claims,
wherein the molar ratio of the tertiary vinyl carbinol of the general formula (I) to the isopropenyl alkyl ether of the general formula (II) ranges from 1:7 to 1:2, preferably ranges from 1:5 to 1:1.5, more preferably ranges from 1:3.5 to 1:2.

5. The process according to any one or more of the preceding claims,
wherein the amount of the catalyst ranges from 0.01-0.3 mol-%, preferably ranges from 0.02-0.25 mol-%, more preferably ranges from 0.1-0.25 mol-%, based on the amount of the tertiary vinyl carbinol of the general formula (I).

6. The process according to any one or more of the preceding claims,
wherein the compound of the general formula (III) is selected from the group consisting of 6-methyl-5-hepten-2-one, 6-methyl-5-octen-2-one, 6,10-dimethyl-5,9-undecadien-2-one, 6,10-dimethyl-5-undecen-2-one and 6,10,14-trimethyl-5,9,13-pentadeca-trien-2-one, preferably wherein the compound of the general formula (III) is 6-methyl-5-hepten-2-one or 6-methyl-5-octen-2-one.

7. Use of a catalyst of the formula (IV) 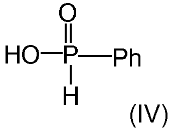 for the manufacture of gamma,delta-unsaturated ketones of the general formula (III) 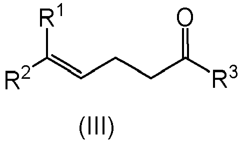 by reacting a tertiary vinyl carbinol of the general formula (I) with an isopropenyl alkyl ether of the general formula (II) 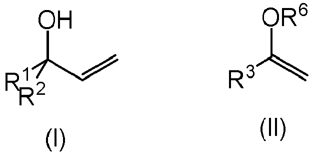 wherein R¹ and R⁶ are independently from each other methyl or ethyl, R³ is methyl, and R² is a saturated or unsaturated linear, branched or cyclic hydrocarbyl group with 1 to 46 C atoms.

8. A reaction mixture comprising a compound of formula (I), a compound of formula (II) and a catalyst of formula (IV) 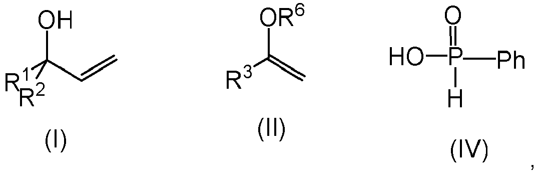 wherein R¹ and R⁶ are independently from each other methyl or ethyl, R³ is methyl, and R² is a saturated or unsaturated linear, branched or cyclic hydrocarbyl group with 1 to 46 C atoms.

9. A reaction mixture consisting essentially of a compound of formula (I), a compound of formula (II) and a catalyst of formula (IV) 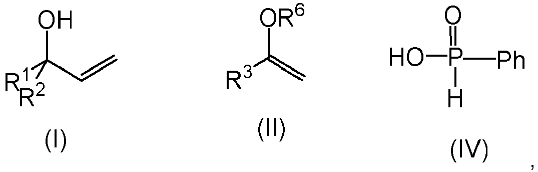 wherein R¹ and R⁶ are independently from each other methyl or ethyl, R³ is methyl, and R² is a saturated or unsaturated linear, branched or cyclic hydrocarbyl group with 1 to 46 C atoms.

## Patentansprüche

1. Verfahren zur Herstellung von gamma,delta-ungesättigten Ketonen der allgemeinen Formel (III) 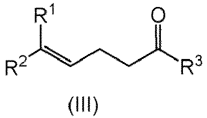 durch Umsetzen eines tertiären Vinylcarbinols der allgemeinen Formel (I) mit einem Isopropenylalkylether der allgemeinen Formel (II) in Gegenwart eines Katalysators der allgemeinen Formel (IV), 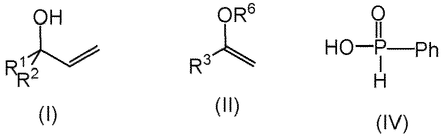 wobei R¹ und R⁶ unabhängig voneinander für Methyl oder Ethyl stehen, R³ für Methyl steht und R² für eine gesättigte oder ungesättigte lineare, verzweigte oder cyclische Hydrocarbylgruppe mit 1 bis 46 C-Atomen steht.

2. Verfahren nach Anspruch 1, wobei die Umsetzung bei einer Temperatur im Bereich von 100 bis 170 °C, vorzugsweise bei einer Temperatur im Bereich von 110 bis 160 °C, weiter bevorzugt bei einer Temperatur im Bereich von 120 bis 150 °C, durchgeführt wird.

3. Verfahren nach einem oder mehreren der vorhergehenden Ansprüche, wobei die Umsetzung bei einem Druck im Bereich von 5 bis 15 bar, vorzugsweise bei einem Druck im Bereich von 8 bis 12 bar, durchgeführt wird.

4. Verfahren nach einem oder mehreren der vorhergehenden Ansprüche, wobei das Molverhältnis von tertiärem Vinylcarbinol der allgemeinen Formel (I) zu Isopropenylalkylether der allgemeinen Formel (II) im Bereich von 1:7 bis 1:2, vorzugsweise im Bereich von 1:5 bis 1:1,5, weiter bevorzugt im Bereich von 1:3,5 bis 1:2, liegt.

5. Verfahren nach einem oder mehreren der vorhergehenden Ansprüche, wobei die Menge des Katalysators im Bereich von 0,01-0,3 Mol-%, vorzugsweise im Bereich von 0,02-0,25 Mol-%, weiter bevorzugt im Bereich von 0,1-0,25 Mol-%, bezogen auf die Menge des tertiären Vinylcarbinols der allgemeinen Formel (I), liegt.

6. Verfahren nach einem oder mehreren der vorhergehenden Ansprüche, wobei die Verbindung der allgemeinen Formel (III) aus der Gruppe bestehend aus 6-Methyl-5-hepten-2-on, 6-Methyl-5-octen-2-on, 6,10-Dimethyl-5,9-undecadien-2-on, 6,10-Dimethyl-5-undecen-2-on und 6,10,14-Trimethyl-5,9,13-penta-decatrien-2-on ausgewählt wird, vorzugsweise wobei es sich bei der Verbindung der allgemeinen Formel (III) um 6-Methyl-5-hepten-2-on oder 6-Methyl-5-octen-2-on handelt.

7. Verwendung eines Katalysators der Formel (IV) 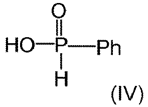 zur Herstellung von gamma,delta-ungesättigten Ketonen der allgemeinen Formel (III) 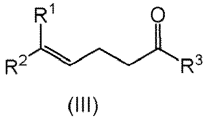 durch Umsetzen eines tertiären Vinylcarbinols der allgemeinen Formel (I) mit einem Isopropenylalkylether der allgemeinen Formel (II) 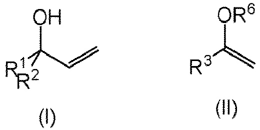 wobei R¹ und R⁶ unabhängig voneinander für Methyl oder Ethyl stehen, R³ für Methyl steht und R² für eine gesättigte oder ungesättigte lineare, verzweigte oder cyclische Hydrocarbylgruppe mit 1 bis 46 C-Atomen steht.

8. Reaktionsmischung, umfassend eine Verbindung der Formel (I), eine Verbindung der Formel (II) und einen Katalysator der Formel (IV) 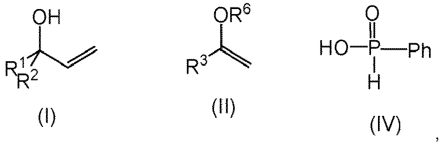 wobei R¹ und R⁶ unabhängig voneinander für Methyl oder Ethyl stehen, R³ für Methyl steht und R² für eine gesättigte oder ungesättigte lineare, verzweigte oder cyclische Hydrocarbylgruppe mit 1 bis 46 C-Atomen steht.

9. Reaktionsmischung, im Wesentlichen bestehend aus einer Verbindung der Formel (I), einer Verbindung der Formel (II) und einem Katalysator der Formel 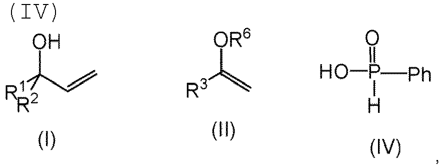 wobei R¹ und R⁶ unabhängig voneinander für Methyl oder Ethyl stehen, R³ für Methyl steht und R² für eine gesättigte oder ungesättigte lineare, verzweigte oder cyclische Hydrocarbylgruppe mit 1 bis 46 C-Atomen steht.

## Revendications

1. Procédé pour la fabrication de cétones gamma,delta-insaturées de la formule générale (III) 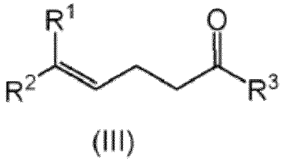 par mise en réaction d'un vinylcarbinol tertiaire de la formule générale (I) avec un éther d'alkyle et d'isoprényle de la formule générale (II) en la présence d'un catalyseur de la formule générale (IV), 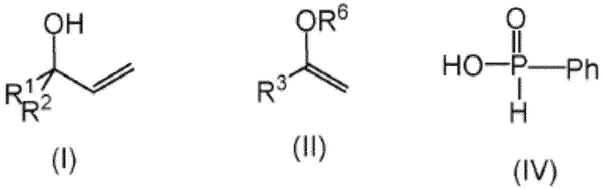 R¹ et R⁶ étant indépendamment l'un de l'autre méthyle ou éthyle, R³ étant méthyle, et R² étant un groupe hydrocarbyle saturé ou insaturé, linéaire, ramifié ou cyclique comportant 1 à 46 atomes de C.

2. Procédé selon la revendication 1, la réaction étant mise en œuvre à une température dans la plage de 100 à 170 °C, préférablement à une température dans la plage de 110 à 160 °C, plus préférablement à une température dans la plage de 120 à 150 °C.

3. Procédé selon l'une quelconque ou plusieurs des revendications précédentes, la réaction étant mise en œuvre à une pression dans la plage de 5 à 15 bars, préférablement à une pression dans la plage de 8 à 12 bars.

4. Procédé selon l'une quelconque ou plusieurs des revendications précédentes, le rapport molaire du vinylcarbinol tertiaire de la formule générale (I) sur l'éther d'alkyle et d'isoprényle de la formule générale (II) se situant dans la plage de 1 : 7 à 1 : 2, préférablement dans la plage de 1 : 5 à 1 : 1,5, plus préférablement dans la plage de 1 : 3,5 à 1 : 2.

5. Procédé selon l'une quelconque ou plusieurs des revendications précédentes, la quantité de catalyseur se situant dans la plage de 0,01 à 0,3 % en moles, préférablement dans la plage de 0,02 à 0,25 % en moles, plus préférablement dans la plage de 0,1 à 0,25 % en moles, sur la base de la quantité du vinylcarbinol tertiaire de la formule générale (I).

6. Procédé selon l'une quelconque ou plusieurs des revendications précédentes, le composé de la formule générale (III) étant choisi dans le groupe constitué par la 6-méthyl-5-heptén-2-one, la 6-méthyl-5-octén-2-one, la 6,10-diméthyl-5,9-undécadién-2-one, la 6,10-diméthyl-5-undécén-2-one et la 6,10,14-triméthyl-5,9,13-pentadéca-trién-2-one, préférablement le composé de formule générale (III) étant la 6-méthyl-5-heptén-2-one ou la 6-méthyl-5-octén-2-one.

7. Utilisation d'un catalyseur de la formule (IV) 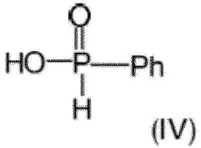 pour la fabrication de de cétones gamma,delta-insaturées de la formule générale (III) 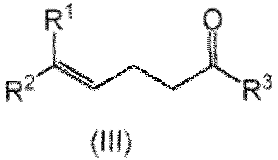 par mise en réaction d'un vinylcarbinol tertiaire de la formule générale (I) avec un éther d'alkyle et d'isoprényle de la formule générale (II) 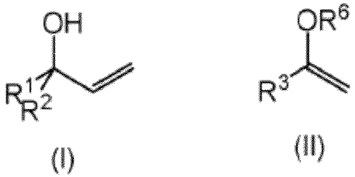 R¹ et R⁶ étant indépendamment l'un de l'autre méthyle ou éthyle, R³ étant méthyle, et R² étant un groupe hydrocarbyle saturé ou insaturé, linéaire, ramifié ou cyclique comportant 1 à 46 atomes de C.

8. Mélange réactionnel comprenant un composé de formule (I), un composé de formule (II) et un catalyseur de formule (IV) 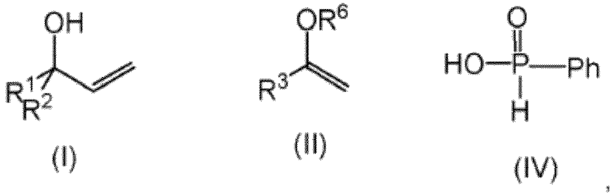 R¹ et R⁶ étant indépendamment l'un de l'autre méthyle ou éthyle, R³ étant méthyle, et R² étant un groupe hydrocarbyle saturé ou insaturé, linéaire, ramifié ou cyclique comportant 1 à 46 atomes de C.

9. Mélange réactionnel essentiellement constitué d'un composé de formule (I), d'un composé de formule (II) et d'un catalyseur de formule (IV) 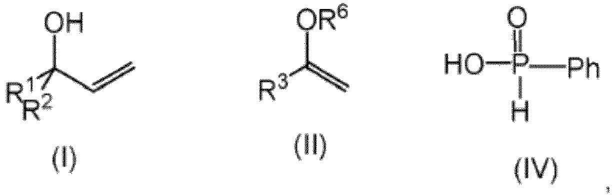 R¹ et R⁶ étant indépendamment l'un de l'autre méthyle ou éthyle, R³ étant méthyle, et R² étant un groupe hydrocarbyle saturé ou insaturé, linéaire, ramifié ou cyclique comportant 1 à 46 atomes de C.
